# EUROPÄISCHE PATENTANMELDUNG

(11) **EP 3 407 357 A1**
(43) Veröffentlichungstag der Anmeldung: **28.11.2018**
(21) Anmeldenummer: 18157444.3
(22) Anmeldetag: 19.02.2018
(51) Int. Cl.: G16H 40/20, G06Q 50/00

(54) **OPTIMIERTE VERTEILUNG VON PERSONEN AN UNTERSUCHUNGSEINRICHTUNGEN**

(71) Anmelder: Siemens Healthcare GmbH, 91052 Erlangen (DE)
(72) Erfinder: Zeller, Mario, 91054 Erlangen (DE)

(57) **Zusammenfassung**

Die Erfindung betrifft ein Verfahren zum automatischen Zuweisen von Untersuchungspersonen zu einer Untersuchungseinrichtung von mehreren medizinischen Untersuchungseinrichtungen durch eine Steuerungseinheit (100), mit den folgenden Schritten:
- Bestimmen von ersten Daten, die personenbezogene Daten sind und nähere Informationen über die Untersuchungsperson aufweisen,
- Bestimmen von zweiten Daten, die Bedienperson-bezogene Daten sind und angeben, wie viele Bedienpersonen an den mehreren medizinischen Untersuchungseinrichtungen arbeiten oder verfügbar sind,
- Bestimmen von dritten Daten, die Untersuchungseinrichtungbezogene Daten sind und Informationen über die Untersuchungseinrichtung selbst aufweisen,
- Zuweisen von Untersuchungspersonen zu einer der Untersuchungseinrichtungen unter Berücksichtigung der ersten Daten, der zweiten Daten und der dritten Daten.

## Beschreibung

Die vorliegende Erfindung betrifft ein Verfahren zum automatischen Zuweisen von Untersuchungspersonen zu einer Untersuchungseinrichtung von mehreren medizinischen Untersuchungseinrichtungen durch eine Steuerungseinheit. Weiterhin wird die zugehörige Steuerungseinheit bereitgestellt sowie ein Computerprogrammprodukt und ein elektronisch lesbarer Datenträger.

Bei medizinischen Untersuchungen, insbesondere bei Untersuchungen mit bildgebenden Vorrichtungen kann es im Notfall von Bedeutung sein, für einen Patienten schnellstmöglich eine Untersuchung an der richtigen Untersuchungseinrichtung durchführen zu können. Beispielsweise ist das Platzieren von adipösen oder multimorbiden Patienten auf einer Untersuchungsliege oft langwierig und herausfordernd wenn die zugehörige bildgebende Vorrichtung beispielsweise nur von einer einzigen Bedienperson belegt ist. Dies kann bei Notfallpatienten zu nicht tolerierbaren Verzögerungen führen. Während der Bildgebungsanteil durch verschiedene Beschleunigungstechniken insbesondere bei MR-Anlagen immer weiter reduziert werden konnte, wird ein nicht unerheblicher Anteil der Untersuchungsdauer bei bildgebenden Systemen durch die Lagerung der Untersuchungsperson und die Vorbereitung der Messung eingenommen. Hierzu zählt auch die Positionierung der notwendigen Lokalspulen, welche zwischen Untersuchungen verschiedener Personen gewechselt werden müssen. Ein optimierter Ablauf bei Untersuchungen an Untersuchungseinrichtungen kann beispielsweise teilweise dadurch erreicht werden, dass nur ähnliche oder gleiche Untersuchungen in einer zeitlichen Abfolge vorgenommen werden. Es ist möglich, ausschließlich oder vorzugsweise Untersuchungen in einem anatomischen Gebiet bei MR-Anlagen in zeitlicher Abfolge zu planen, um Spulenwechselzeiten zu vermeiden. Dies löst jedoch nicht das Problem bei Notfällen.

In der US 7,562,026 B2 wird ein Radiologieinformationssystem bereitgestellt welches eine dynamische Patientenplanung ermöglicht. Jedoch führt auch dies nicht zu einem befriedigenden Ergebnis.

Es ist somit eine Aufgabe der vorliegenden Erfindung, ein Verfahren zum automatischen Zuweisen von Untersuchungspersonen zu Untersuchungseinrichtungen zu verbessern.

Diese Aufgabe wird durch die Merkmale der unabhängigen Ansprüche gelöst. In den abhängigen Ansprüchen sind weitere Ausführungsformen beschrieben.

Gemäß einem ersten Aspekt wird ein Verfahren zum automatischen Zuweisen von Untersuchungspersonen zu einer Untersuchungseinrichtung von mehreren medizinischen Untersuchungseinrichtungen bereitgestellt, wobei das Verfahren durch eine Steuerungseinheit durchgeführt wird. Es werden erste Daten bestimmt, die personenbezogene Daten sind und nähere Informationen über die Untersuchungsperson aufweisen. Weiterhin werden zweite Daten bestimmt, die Bedienperson bezogene Daten sind und zumindest angeben, wie viele Bedienpersonen an den mehreren medizinischen Untersuchungseinrichtungen arbeiten oder verfügbar sind. Weiterhin werden dritte Daten bestimmt, die Untersuchungseinrichtung bezogene Daten sind und Informationen und über die Untersuchungseinrichtung selbst aufweisen. Anschließend ist es möglich die Untersuchungspersonen zu einer der Untersuchungseinrichtungen zuzuweisen unter Berücksichtigung der ersten Daten, der zweiten Daten und der dritten Daten.

Durch die Verwendung der verschiedenen Daten, nämlich die Berücksichtigung der personenbezogenen Daten der Untersuchungsperson, die Berücksichtigung der Bedienungsperson bezogenen Daten und der Untersuchungseinrichtung bezogenen Daten ist es möglich, die optimale Untersuchungseinrichtungen für eine Untersuchungsperson auszuwählen und sie dieser Untersuchungseinrichtung zuzuweisen. Mit diesen drei verschiedenen Arten von Daten ist ein umfassender Überblick möglich, welche Untersuchungseinrichtungen der verschiedenen Untersuchungseinrichtungen die beste ist für eine zu untersuchende Person. Insbesondere bei einer zeitlichen Dringlichkeit bei Notfällen kann die optimale Untersuchungseinrichtung ausgewählt werden, beispielsweise diejenige, bei der für die vorzunehmende Untersuchung das notwendige Bedienpersonal vorhanden ist. Die möglichen Untersuchungseinrichtungen sind von der gleichen Art, sie können sich jedoch in Ausstattung, Performance etc. unterscheiden. Die Untersuchungseinrichtungen können alle MR-Anlagen sein, jedoch kann es sich um Anlagen mit verschiedenen Bauformen der Magnete, mit verschiedenen Feldstärken oder Ausstattungen handeln. Es können auch verschiedene CT, PET oder sonstige bildgebende Vorrichtungen sein, oder Operationstische mit unterschiedlicher Ausstattung.

Beispielsweise ist es möglich, die zweiten Daten, nämlich die Bedienperson bezogenen Daten aus Dienstplänen der möglichen Bedienpersonen zu gewinnen, wobei die zweiten Daten tagesaktuelle Daten aufweisen, welche angeben welche und wie viele Bedienpersonen im Moment an den einzelnen Untersuchungseinrichtungen verfügbar sind, wobei die zweiten Daten ebenso zukünftige Daten aufweisen, die angeben welche und wie viele Personen in Zukunft, beispielsweise in einem Zeitabschnitt von Tagen, Wochen, oder Monaten an den einzelnen Versuchseinrichtungen verfügbar sind. Das Zuweisen von Untersuchungspersonen erfolgt dann unter Berücksichtigung von den tagesaktuellen oder zukünftigen Daten.

Weiterhin ist es möglich, dass die ersten Daten, die personenbezogenen Daten eine der folgenden personenbezogenen Daten aufweisen. Eine erste Information, die in den ersten Daten enthalten ist kann einen Information über die Dringlichkeit der vorzunehmenden Untersuchung sein. Eine weitere Information in den ersten Daten kann ein Gewicht der Untersuchungsperson sein, die untersucht wird. Weiterhin kann eine Information über die Größe der Untersuchungsperson vorhanden sein in den ersten Daten. Zusätzlich können Informationen über mögliche Allergien bei Untersuchungsperson oder beispielsweise eine Kontrastmittelunverträglichkeit vorhanden sein in den ersten Daten. Die ersten Daten können weiterhin Informationen über den zu untersuchenden anatomischen Bereich aufweisen, eine Information darüber, ob Kontrastmittel bei der Untersuchung notwendig sein wird, oder eine Information ob die Untersuchungsperson beispielsweise klaustrophob ist. Von diesen Informationsstücken können einige vorhanden sein und verwendet werden als erste Daten bei der Zuweisung zu den unterschiedlichen Untersuchungseinrichtungen.

Diese ersten Daten können beispielsweise mithilfe einer Patientendatenbank vorhanden sein die in der Einrichtung wie beispielsweise dem Krankenhaus vorhanden ist, in dem die mehreren Untersuchungseinrichtungen stehen.

Eine weitere Möglichkeit die zweiten Daten, die Bedienperson bezogenen Daten zu erfassen besteht darin, dass diese zweiten Daten aus Anmeldedaten gewonnen werden, mit denen sich die Bedienperson an den medizinischen Untersuchungseinrichtungen zur Eigenidentifikation anmelden. Diese Informationen können an den einzelnen Untersuchungseinrichtungen auch mithilfe von RFID-Vorrichtung gewonnen werden mit der sich eine Bedienperson an den Untersuchungseinrichtungen identifiziert. Dadurch können aktuelle Belegungen der Anlagen mit Bedienpersonal bestimmt werden.

Die Untersuchungseinrichtung kann eine bildgebende Vorrichtung sein, beispielsweise eine MR-Anlage, jedoch ist es auch möglich, dass es sich um eine CT-Anlage oder um jede andere bildgebende Vorrichtung handelt. Die dritten Daten, welche Informationen zur Untersuchungseinrichtung bereitstellen, können auch aus den erzeugten Bilddaten von Untersuchungspersonen bestimmt werden, die zuletzt in der bildgebenden Vorrichtung untersucht wurden. Insbesondere bei MR-Anlagen, lassen sich darüber beispielsweise Informationen gewinnen über eine Tischpositionen, die aktuell verwendeten Spulen, die aktuell verwendete Untersuchungsart, die Restdauer der Untersuchung, den Bohrungsdurchmesser der MR-Anlage, die Verfügbarkeit von verschiedenen Spulen etc.

Beispielsweise ist es möglich, dass die verschiedenen Untersuchungspersonen den verschiedenen MR-Anlagen derart zugewiesen werden, dass bei zumindest einer der MR-Anlage eine Anzahl der Wechsel der Empfangsspulen bei der Untersuchung von verschiedenen Untersuchungspersonen minimiert wird.

Bei MR-Anlagen können die dritten Daten auch dazu verwendet werden, eine Information über die Größe der Bohrung der MR-Anlage zu erhalten, so dass Untersuchungspersonen mit Klaustrophobie beispielsweise der MR-Anlage zugewiesen wird, die eine größere Bohrung hat als andere MR-Anlagen. Dies gilt ebenso für adipöse Untersuchungspersonen.

Die Erfindung ist jedoch nicht auf MR-Anlagen oder medizinische bildgebende Vorrichtungen beschränkt. Die Untersuchungseinrichtung kann auch auf den interventionellen Bereich ausgeweitet werden, wobei die Untersuchungseinrichtung dann etwa eine OP-Einrichtung oder eine Herzkatheter-Einrichtung ist.

Die Steuerungseinrichtung kann auch Zugriff auf Trainingsdaten haben, die Auskunft darüber geben, mit welchen 1., 2. und 3. Daten aus bisherigen Untersuchungen von verschiedenen Personen die verschiedenen Untersuchungspersonen auf die Untersuchungseinrichtung verteilt wurden. Die Steuerungseinheit kann dann anhand dieser Trainingsdaten die Zuweisung von Untersuchungspersonen zu einer Untersuchungseinrichtung lernen.

Weiterhin kann das Zuweisen von Untersuchungspersonen zu einer der Untersuchungseinrichtungen unter Verwendung von voreingestellten Regeln erfolgen, die Informationen enthalten welche Untersuchungseinrichtungen eine Untersuchungsperson in Abhängigkeit von den 1., 2. und 3. Daten zugewiesen wird. Mit diesen voreingestellten Regeln ist es möglich für die Betreiber der Untersuchungseinrichtungen festzulegen, wie die Verteilung auf die einzelnen Untersuchungseinrichtungen erfolgen soll.

Weiterhin wird die zugehörige Steuerungseinheit bereitgestellt, die ausgebildet ist zum automatischen Zuweisen von Untersuchungspersonen zu einer Untersuchungseinrichtung von mehreren medizinischen Untersuchungseinrichtungen. Die Steuerungseinheit weist eine Speichereinheit und zumindest eine Prozessoreinheit auf, die in der Speichereinheit gespeicherte Steuerungsbefehle ausführen kann. Die Steuerungseinheit ist hierbei bei Ausführung der Steuerbefehle in der Prozessoreinheit ausgebildet, das oben beschriebene Verfahren durchzuführen.

Weiterhin wird ein Computerprogrammprodukt bereitgestellt, welches Steuerungsbefehle umfasst und direkt in eine Speichereinheit einer programmierbaren Steuerungseinheit ladbar ist, um die Schritte des oben beschriebenen Verfahrens auszuführen, wenn die Steuerungsbefehle in der Steuerungseinheit ausgeführt werden.

Zusätzlich wird ein elektronisch lesbarer Datenträger mit darauf gespeicherten elektronisch lesbaren Steuerungsbefehlen bereitgestellt, wobei die Steuerungsbefehle derart ausgebildet sind, dass sie bei Verwendung des Datenträgers in einer Steuerungseinheit das Verfahren wie oben beschrieben durchführen.

Die oben dargelegten Merkmale und Merkmale, die nachfolgend beschrieben werden, können nicht nur in den entsprechend explizit dargelegten Kombinationen verwendet werden, sondern auch in anderen Kombinationen, sofern es nicht explizit anders erwähnt ist, ohne den Schutzumfang der vorliegenden Erfindung zu verlassen.

Die Erfindung wird unter Bezugnahme auf die beiliegenden Zeichnungen näher erläutert.
Figur 1 zeigt schematisch eine Steuerungseinheit, die ausgebildet ist automatisch Untersuchungspersonen zu verschiedenen Untersuchungseinrichtungen zuzuweisen.
Figur 2 zeigt ein Flussdiagramm mit den Schritten zum Zuweisen der Untersuchungspersonen zu den Untersuchungseinrichtungen.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Weiterhin sind die Figuren schematische Darstellungen verschiedener Ausführungsformen. Die in den Figuren dargestellten Elemente sind nicht notwendiger Weise maßstabsgetreu dargestellt. Vielmehr sind die in den Figuren dargestellten Elemente derart wiedergegeben, dass ihre Funktion und der Zweck für den Fachmann verständlich werden. Die in den Figuren dargestellten Verbindungen zwischen funktionellen Einheiten oder sonstigen Elementen können auch als indirekte Verbindung implementiert werden, wobei eine Verbindung drahtlos oder drahtgebunden sein kann. Funktionelle Einheiten können als Hardware, Software, Firmware oder eine Kombination daraus implementiert werden.

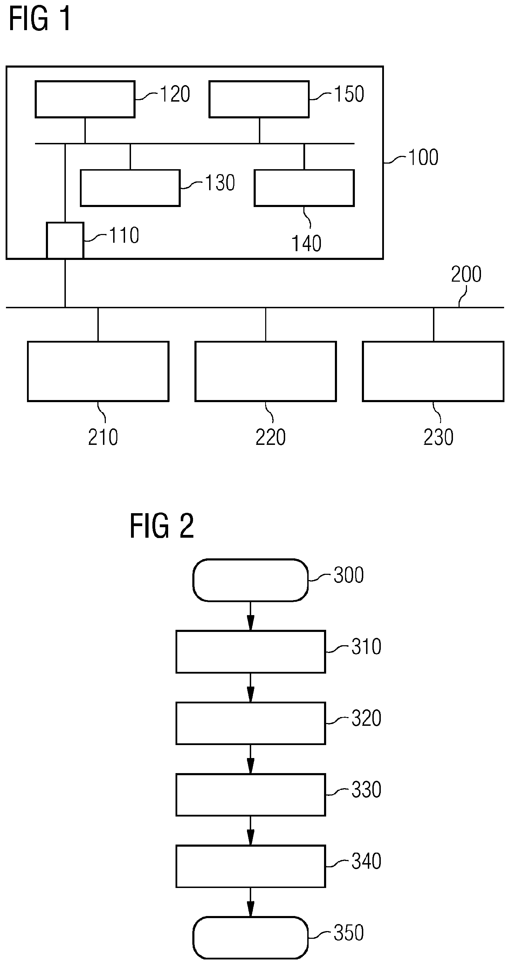

In Figur 1 ist schematisch eine Steuerungseinheit 100 dargestellt, die automatisch Untersuchungspersonen oder Patienten zu einer von mehreren medizinischen Untersuchungseinrichtungen zuweisen kann. Die Steuerungseinheit 100 weist eine Schnittstelle 110 auf, mit der eine Kommunikation mit anderen Einheiten ermöglicht wird, beispielsweise mit einer ersten Datenbank 210 einer zweiten Datenbank 220 oder einer dritten Datenbank 230, die später noch im Detail erläutert werden. Über die Schnittstelle 110 werden Daten oder Steuerbefehle empfangen oder ausgesendet. Weiterhin weist die Steuerungseinheit 100 eine Prozessoreinheit 120 auf, die einen oder mehrere Prozessoren aufweist, die Steuerungsbefehle ausführen können, die beispielsweise in einer Speichereinheit 130 gespeichert sind. Die Steuerungseinheit 100 kann weiterhin eine Eingabeeinheit 140 und eine Anzeigeeinheit 150 aufweisen. Über die Eingabeeinheit 140 kann eine Bedienperson der Steuerungseinheit 100 diese bedienen und über die Anzeigeeinheit 150 können Informationen angezeigt werden, beispielsweise die bestimmte Zuordnung zu den einzelnen Untersuchungseinrichtungen. Die Steuerungseinheit 100 ist über ein Bussystem 200 mit der ersten Datenbank 210 verbunden, die erste Daten aufweist, die Personenbezogene Daten sind und nähere Informationen über die Untersuchungsperson enthalten. Die erste Datenbank 210 kann beispielsweise ein Krankenhausinformationssystem HIS, KIS (Hospital Information System, Krankenhausinformationssystem) sein. Zusätzlich kann die Steuerungseinheit 100 auf eine zweite Datenbank 220 zugreifen, über die Informationen über die Bedienpersonen erhalten werden können, die an den einzelnen Untersuchungseinrichtungen tätig sind. Die Untersuchungseinrichtungen können bildgebende Vorrichtungen sein, wie MR-Anlagen, Computertomografen, PET-Systeme, oder die Untersuchungseinrichtungen können interventionelle Anlagen sein wie OP-Anlagen oder Herzkatheter-Vorrichtungen. Die zweite Datenbank 220 kann beispielsweise die Dienstpläne der einzelnen Bedienpersonen und diensthabenden Ärzte für die einzelnen Untersuchungseinrichtungen beinhalten. Weiterhin ist es möglich, dass die zweite Datenbank 220 Informationen aus einem Anmeldesystem bezieht, mit welchem sich die einzelnen Bedienpersonen an den Untersuchungseinrichtungen anmelden, beispielsweise über RFID-Tags oder Ähnliches. Weiterhin ist die dritte Datenbank 230 vorhanden, die Untersuchungseinrichtungsbezogene Daten aufweist. Diese Daten lassen sich beispielsweise über eine hierfür vorgesehene Schnittstelle gewinnen, die in einer Untersuchungseinrichtung selbst vorhanden ist, oder es können im Falle von einer bildgebenden Vorrichtungen die in den Bildern gespeicherten Informationen wie beispielsweise die Metainformationen, der Header, untersucht werden zur Bestimmung von Informationen über den aktuellen Status der bildgebenden Vorrichtung. Da diese Daten in einem mehr oder weniger normierten Format vorliegen beispielweise in einem DICOM-Format, ist es einfach, aus diesen Daten die notwendige Information zu gewinnen.

Bei dem in Figur 1 gezeigten Beispiel sind die drei Datenbanken 210, 220 und 230 getrennte Datenbanken. Selbstverständlich können diese ersten, zweiten und dritten Daten auch in einer einzigen Datenbank gespeichert sein, und es ist möglich, dass die darin enthaltenen Daten auch in der Steuerungseinheit selbst gespeicherten sind, beispielsweise im der Speichereinheit 130.

Die in Figur 1 gezeigte Vorrichtung hat nun Zugriff auf eine Vielzahl von Daten wie beispielsweise die aktuelle Belegung des Bedienpersonals an den einzelnen Untersuchungseinrichtungen. Es liegt ein Information über den aktuellen Status und die Grunddaten der Untersuchungseinrichtung vor, im Falle einer MR-Anlage beispielsweise die Tischposition, die aktuell verwendeten Spulen, die aktuelle Untersuchungsart, die Restdauer der Untersuchung, der Durchmesser der Bohrung, die mögliche Tischverfahrbarkeit, die Spulenverfügbarkeit etc. Weiterhin sind Daten über die Untersuchungsperson vorhanden, welche untersucht werden soll. Diese Daten können beispielsweise Gewicht, Größe der Untersuchungsregion, Kontrastmittelunverträglichkeit, Klaustrophobieanfälligkeit enthalten oder Informationen ob beispielsweise Kontrastmittel erforderlich ist, und wie dringend die notwendige Untersuchung ist.

Basierend auf diesen Daten erfolgt dann regelbasiert aufgrund von Regeln, die in der Speichereinheit 130 gespeichert sind oder durch ein machine learning-Verfahren, welches beispielweise anhand von Trainingsdatensätzen vergangener Untersuchungen eine optimale Patientenzuordnung erlernt hat, eine Zuweisung der Untersuchungspersonen an die verschiedenen Untersuchungseinrichtungen erfolgen. Sind beispielsweise zwei, drei oder vier verschiedene MR-Anlagen vorhanden, so können Untersuchungen ähnlicher Körperregionen mit wenigen Spulenwechseln an einer MR-Anlage zusammengeführt werden, um ein häufiges Spulenwechseln zu vermeiden. Weiterhin ist es dadurch möglich, adipöse oder klaustrophobe Untersuchungspersonen den MR-Anlagen zuzuweisen, die einen größeren Bohrungsdurchmesser haben als andere MR-Anlagen. Dies kann eine zeitaufwendige Neuplanung vermeiden, falls der Untersuchungsperson eine Untersuchung verweigert oder nicht möglich ist. Weiterhin ist es möglich, Untersuchungen für Personen, welche mehr Zeit erfordern, weil sie entweder adipös sind oder einen sehr schlechten Gesamtgesundheitszustand haben, an den Untersuchungseinrichtungen zu planen, an denen mehr Bedienpersonal verfügbar ist. Dringende Untersuchungen können an einem aktuell verfügbaren oder leerstehenden Gerät vorgenommen werden und eventuell nachfolgende Untersuchungen können ebenfalls neu verteilt werden. Zusätzlich ist ein manuelles Umplanen von Untersuchungspersonen weiterhin möglich, beispielsweise über die Eingabe 140. Da in der Speichereinheit 130 auch benutzerdefinierte Regeln ablegbar sind, können diese bei dem automatischen Zuweisen durch die Steuerungseinheit 100 berücksichtigt werden. Das in Figur 1 gezeigte System kann auch direkt über dem Bus 200 mit den einzelnen Untersuchungseinrichtungen verbunden sein, um von dort entweder Informationen über die Untersuchungseinrichtungen zu bekommen oder um die berechnete Zuweisung der Untersuchungspersonen den Untersuchungseinrichtungen zu melden.

In Figur 2 ist das Verfahren schematisch zusammengefasst. Das Verfahren startet in einem Schritt 300 und in einem Schritt 310 erfolgt das Bestimmen der ersten Daten, die die personenbezogenen Daten sind und nähere Informationen über die Untersuchungsperson aufweisen, wobei diese Daten aus der ersten Datenbank 210 gewonnen werden. In einem weiteren Schritt 320 werden die Bedienperson-bezogenen Daten bestimmt, wodurch bestimmt wird, wie viele Bedienpersonen oder welche Bedienpersonen an den einzelnen Untersuchungseinrichtungen verfügbar sind. In einem dritten Schritt 330 werden die Untersuchungseinrichtungs-bezogenen Daten bestimmt aus der Datenbank 320 oder direkt über die Untersuchungseinrichtungen selbst. Mit diesen drei Daten ist es dann im Schritt 340 möglich, eine Untersuchungsperson zu einer der Untersuchungseinrichtungen optimal zuzuweisen. Das Verfahren endet im Schritt 350.

Das oben beschriebene Verfahren ermöglicht eine intelligente Verteilung von Untersuchungspersonen auf die zugehörigen Untersuchungseinrichtungen unter Einbeziehung aller notwendigen Daten. Es hat den Vorteil, dass im Notfall die Untersuchungsperson an die richtige Untersuchungseinrichtung zugewiesen wird. Weiterhin ist ein erhöhter Durchsatz möglich durch weniger neue Planungen und einen optimalen Einsatz der Bedienpersonen. Dies führt auch zu einer erhöhten Zufriedenheit bei den Bedienpersonen und bei den untersuchten Personen aufgrund der kürzeren Wartezeit, der richtigen Untersuchungseinrichtung. Zusätzlich wird das Planungspersonal entlastet, da weniger manuelle Planungen durchgeführt werden müssen.

## Patentansprüche

1. Verfahren zum automatischen Zuweisen von Untersuchungspersonen zu einer Untersuchungseinrichtung von mehreren medizinischen Untersuchungseinrichtungen durch eine Steuerungseinheit (100), mit den folgenden Schritten:
- Bestimmen von ersten Daten, die personenbezogene Daten sind und nähere Informationen über die Untersuchungsperson aufweisen,
- Bestimmen von zweiten Daten, die Bedienperson-bezogene Daten sind und angeben, wie viele Bedienpersonen an den mehreren medizinischen Untersuchungseinrichtungen arbeiten oder verfügbar sind,
- Bestimmen von dritten Daten, die Untersuchungseinrichtungbezogene Daten sind und Informationen über die Untersuchungseinrichtung selbst aufweisen,
- Zuweisen von Untersuchungspersonen zu einer der Untersuchungseinrichtungen unter Berücksichtigung der ersten Daten, der zweiten Daten und der dritten Daten.

2. Verfahren nach Anspruch 1, **dadurch gekennzeichnet, dass** die zweiten Daten aus Dienstplänen der möglichen Bedienpersonen gewonnen werden, wobei die zweiten Daten tagesaktuelle Daten, die angeben, welche und wie viele Bedienpersonen im Moment an den einzelnen Untersuchungseinrichtungen verfügbar sind, und zukünftige Daten aufweisen, die angeben, welche und wie viele Bedienpersonen in Zukunft an den einzelnen Untersuchungseinrichtungen verfügbar sind, wobei das Zuweisen von Untersuchungspersonen unter Berücksichtigung von den tagesaktuellen und den zukünftigen Daten erfolgt.

3. Verfahren nach Anspruch 1 oder 2, **dadurch gekennzeichnet, dass** die ersten Daten zumindest eine der folgenden personenbezogenen Informationen aufweisen: eine Information über das Gewicht der Untersuchungsperson, eine Information über die Größe der Untersuchungsperson, eine Information über eine Kontrastmittelunverträglichkeit der Untersuchungsperson, eine Information über den bei der Untersuchungsperson zu untersuchenden anatomischen Bereich, eine Information über eine Priorität der durchzuführenden Untersuchung, eine Information, ob bei der durchzuführenden Untersuchung Kontrastmittel notwendig ist, eine Information ob die Untersuchungsperson klaustrophob ist, eine Information welche Allergien bei der Untersuchungsperson vorliegen.

4. Verfahren nach einem der vorstehenden Ansprüche, **dadurch gekennzeichnet, dass** die ersten Daten mit Hilfe einer Patientendatenbank einer Einrichtung bestimmt werden, in der die mehreren Untersuchungseinrichtungen vorhanden sind.

5. Verfahren nach einem der vorstehenden Ansprüche, **dadurch gekennzeichnet, dass** die zweiten Daten aus Anmeldedaten bestimmt werden, mit denen sich die Bedienpersonen jeweils an der medizinischen Untersuchungseinrichtung zur eigenen Identifikation anmelden.

6. Verfahren nach einem der vorstehenden Ansprüche, **dadurch gekennzeichnet, dass** die ersten Daten Informationen über ein Gewicht der Untersuchungsperson aufweisen, wobei das Zuweisen zu einer Untersuchungseinrichtung unter Berücksichtigung des Gewichts der Untersuchungsperson erfolgt.

7. Verfahren nach einem der vorstehenden Ansprüche, **dadurch gekennzeichnet, dass** die Untersuchungseinrichtung eine bildgebende Vorrichtung ist, wobei die dritten Daten bestimmt werden aus Metainformationen, die zusammen mit den zuvor erzeugten Bilddaten von Untersuchungspersonen gespeichert sind, die in der bildgebenden Vorrichtung untersucht wurden.

8. Verfahren nach einem der vorstehenden Ansprüche, **dadurch gekennzeichnet, dass** die Untersuchungseinrichtungen MR Anlagen sind, wobei die dritten Daten zumindest eine der folgenden zusätzlichen Informationen aufweisen: eine Verfügbarkeit verschiedener Empfangsspulen zum Empfangen von MR Signalen, eine Tischposition einer Untersuchungsliege, auf der die Untersuchungsperson während der Untersuchung liegt, die aktuell verwendete zumindest eine Empfangsspule zum Empfangen von MR Signalen, die Art der momentan durchgeführten MR Messung, der verbleibenden Untersuchungsdauer der momentan durchgeführten MR Messung, eine Größe des in der MR Anlage zur Verfügung stehenden Raums zur Lagerung der Untersuchungsperson.

9. Verfahren nach Anspruch 8, **dadurch gekennzeichnet, dass** verschiedene Untersuchungspersonen den verschiedenen MR Anlagen derart zugewiesen werden, dass bei zumindest einer der MR Anlagen eine Anzahl der Wechsel der Empfangsspulen bei der Untersuchung von verschiedenen Untersuchungspersonen minimiert wird.

10. Verfahren nach Anspruch 8 oder 9, **dadurch gekennzeichnet, dass** die Untersuchungspersonen mit Klaustrophobie oder mit einem Gewicht größer als ein Grenzwert unter den mehreren MR Anlagen der MR Anlage zugewiesen wird, die einen größeren Raum zur Lagerung der Untersuchungsperson hat als andere der mehreren MR Anlagen.

11. Verfahren nach einem der vorstehenden Ansprüche, **dadurch gekennzeichnet, dass** die Steuerungseinheit Zugriff auf Trainingsdaten hat, die Auskunft darüber geben mit welchen ersten, zweiten und dritten Daten aus bisherigen Untersuchungen von verschiedenen Untersuchungspersonen die verschiedenen Untersuchungspersonen auf die Untersuchungseinrichtungen verteilt wurden, wobei die Steuerungseinheit unter Verwendung der Trainingsdaten die Untersuchungspersonen einer der mehreren Untersuchungseinrichtungen zuweist.

12. Verfahren nach einem der vorstehenden Ansprüche, **dadurch gekennzeichnet, dass** das Zuweisen von Untersuchungspersonen zu einer der Untersuchungseinrichtungen unter Verwendung von voreingestellten Regeln erfolgt, die Informationen enthalten, welcher Untersuchungseinrichtung eine Untersuchungsperson in Abhängigkeit von den ersten, zweiten und dritten Daten zugewiesen wird.

13. Steuerungseinheit (100), die ausgebildet ist, zum automatischen Zuweisen von Untersuchungspersonen zu einer Untersuchungseinrichtung von mehreren medizinischen Untersuchungseinrichtungen, wobei die Steuerungseinheit (100) eine Speichereinheit (130) und zumindest eine Prozessoreinheit (120) aufweist, die in der Speichereinheit gespeicherte Steuerungsbefehle ausführen kann, wobei die Steuerungseinheit (100) bei Ausführen der Steuerungsbefehle in der Prozessoreinheit (120) ausgebildet ist zum Durchführen eines Verfahrens nach einem der Ansprüche 1 bis 12.

14. Computerprogrammprodukt, welches Steuerungsbefehle umfasst und direkt in eine Speichereinheit einer programmierbaren Steuerungseinheit ladbar ist, um alle Schritte des Verfahrens nach einem der Ansprüche 1 bis 12 auszuführen, wenn die Steuerungsbefehle in der Steuerungseinheit ausgeführt werden.

15. Elektronisch lesbarer Datenträger mit darauf gespeicherten elektronisch lesbaren Steuerungsbefehlen, welche derart ausgestaltet sind, dass sie bei Verwendung des Datenträgers in einer Steuerungseinheit das Verfahren nach einem der Ansprüche 1 bis 12 durchführen.

## Geänderte Patentansprüche

### Geänderte Patentansprüche gemäss Regel 137(2) EPÜ.

1. Verfahren zum automatischen Zuweisen von Untersuchungspersonen zu einer MR-Anlage von mehreren MR-Anlagen durch eine Steuerungseinheit (100), mit den folgenden Schritten:
- Bestimmen von ersten Daten, die personenbezogene Daten sind und nähere Informationen über die Untersuchungsperson aufweisen,
- Bestimmen von zweiten Daten, die Bedienperson-bezogene Daten sind und angeben, wie viele Bedienpersonen an den mehreren MR Anlagen arbeiten oder verfügbar sind, wobei die zweiten Daten aus Dienstplänen der möglichen Bedienpersonen gewonnen werden, wobei die zweiten Daten tagesaktuelle Daten, die angeben, welche und wie viele Bedienpersonen im Moment an den einzelnen MR Anlagen verfügbar sind, und zukünftige Daten aufweisen, die angeben, welche und wie viele Bedienpersonen in Zukunft an den einzelnen MR Anlagen verfügbar sind,
- Bestimmen von dritten Daten, die MR Anlage-bezogene Daten sind und Informationen über die MR Anlage selbst aufweisen, mit der Information über die aktuell verwendete zumindest eine Empfangsspule zum Empfangen von MR Signalen,
- Zuweisen von Untersuchungspersonen zu einer der MR Anlagen unter Berücksichtigung der ersten Daten, der zweiten Daten und der dritten Daten, wobei das Zuweisen von Untersuchungspersonen unter Berücksichtigung von den tagesaktuellen und den zukünftigen Daten erfolgt, wobei verschiedene Untersuchungspersonen den verschiedenen MR Anlagen derart zugewiesen werden, dass bei zumindest einer der MR Anlagen eine Anzahl der Wechsel der Empfangsspulen bei der Untersuchung von verschiedenen Untersuchungspersonen minimiert wird.

2. Verfahren nach Anspruch 1, **dadurch gekennzeichnet, dass** die ersten Daten zumindest eine der folgenden personenbezogenen Informationen aufweisen: eine Information über das Gewicht der Untersuchungsperson, eine Information über die Größe der Untersuchungsperson, eine Information über eine Kontrastmittelunverträglichkeit der Untersuchungsperson, eine Information über den bei der Untersuchungsperson zu untersuchenden anatomischen Bereich, eine Information über eine Priorität der durchzuführenden Untersuchung, eine Information, ob bei der durchzuführenden Untersuchung Kontrastmittel notwendig ist, eine Information ob die Untersuchungsperson klaustrophob ist, eine Information welche Allergien bei der Untersuchungsperson vorliegen.

3. Verfahren nach einem der vorstehenden Ansprüche, **dadurch gekennzeichnet, dass** die ersten Daten mit Hilfe einer Patientendatenbank einer Einrichtung bestimmt werden, in der die mehreren MR Anlagen vorhanden sind.

4. Verfahren nach einem der vorstehenden Ansprüche, **dadurch gekennzeichnet, dass** die zweiten Daten aus Anmeldedaten bestimmt werden, mit denen sich die Bedienpersonen jeweils an der MR Anlage zur eigenen Identifikation anmelden.

5. Verfahren nach einem der vorstehenden Ansprüche, **dadurch gekennzeichnet, dass** die ersten Daten Informationen über ein Gewicht der Untersuchungsperson aufweisen, wobei das Zuweisen zu einer Untersuchungseinrichtung unter Berücksichtigung des Gewichts der Untersuchungsperson erfolgt.

6. Verfahren nach einem der vorstehenden Ansprüche, **dadurch gekennzeichnet, dass** die dritten Daten bestimmt werden aus Metainformationen, die zusammen mit den zuvor erzeugten Bilddaten von Untersuchungspersonen gespeichert sind, die in der MR-Anlage untersucht wurden.

7. Verfahren nach Anspruch 1, **dadurch gekennzeichnet, dass** die Untersuchungspersonen mit Klaustrophobie oder mit einem Gewicht größer als ein Grenzwert unter den mehreren MR Anlagen der MR Anlage zugewiesen wird, die einen größeren Raum zur Lagerung der Untersuchungsperson hat als andere der mehreren MR Anlagen.

8. Verfahren nach einem der vorstehenden Ansprüche, **dadurch gekennzeichnet, dass** die Steuerungseinheit Zugriff auf Trainingsdaten hat, die Auskunft darüber geben mit welchen ersten, zweiten und dritten Daten aus bisherigen Untersuchungen von verschiedenen Untersuchungspersonen die verschiedenen Untersuchungspersonen auf die Untersuchungseinrichtungen verteilt wurden, wobei die Steuerungseinheit unter Verwendung der Trainingsdaten die Untersuchungspersonen einer der mehreren Untersuchungseinrichtungen zuweist.

9. Verfahren nach einem der vorstehenden Ansprüche, **dadurch gekennzeichnet, dass** das Zuweisen von Untersuchungspersonen zu einer der Untersuchungseinrichtungen unter Verwendung von voreingestellten Regeln erfolgt, die Informationen enthalten, welcher Untersuchungseinrichtung eine Untersuchungsperson in Abhängigkeit von den ersten, zweiten und dritten Daten zugewiesen wird.

10. Steuerungseinheit (100), die ausgebildet ist, zum automatischen Zuweisen von Untersuchungspersonen zu einer Untersuchungseinrichtung von mehreren medizinischen Untersuchungseinrichtungen, wobei die Steuerungseinheit (100) eine Speichereinheit (130) und zumindest eine Prozessoreinheit (120) aufweist, die in der Speichereinheit gespeicherte Steuerungsbefehle ausführen kann, wobei die Steuerungseinheit (100) bei Ausführen der Steuerungsbefehle in der Prozessoreinheit (120) ausgebildet ist zum Durchführen eines Verfahrens nach einem der Ansprüche 1 bis 9.

11. Computerprogrammprodukt, welches Steuerungsbefehle umfasst und direkt in eine Speichereinheit einer programmierbaren Steuerungseinheit ladbar ist, um alle Schritte des Verfahrens nach einem der Ansprüche 1 bis 9 auszuführen, wenn die Steuerungsbefehle in der Steuerungseinheit ausgeführt werden.

12. Elektronisch lesbarer Datenträger mit darauf gespeicherten elektronisch lesbaren Steuerungsbefehlen, welche derart ausgestaltet sind, dass sie bei Verwendung des Datenträgers in einer Steuerungseinheit das Verfahren nach einem der Ansprüche 1 bis 9 durchführen.
